(19) Europäisches Patentamt
European Patent Office
Office européen des brevets

(11) Publication number: **0 034 413 A1**

(12) # EUROPEAN PATENT APPLICATION

(21) Application number: 81300284.7

(22) Date of filing: 22.01.81

(51) Int. Cl.[3]: C 07 C 65/24
C 07 C 51/265, B 01 J 23/16
B 01 J 23/74, B 01 J 27/08

(30) Priority: 12.02.80 GB 8004607

(43) Date of publication of application: 26.08.81 Bulletin 81/34

(84) Designated Contracting States: BE CH DE FR GB IT LI NL

(71) Applicant: IMPERIAL CHEMICAL INDUSTRIES LIMITED
Imperial Chemical House Millbank
London SW1P 3JF(GB)

(72) Inventor: Kaye, Albert Edward
23 Meadow Road
Middleton Manchester M24 1WH(GB)

(72) Inventor: Morley, John Oswald
9 Winchester Close
Bamford Rochdale Lancashire(GB)

(72) Inventor: Tucker, Alan Cyril
29 High Meadows
Bromley Cross Nr. Bolton Lancashire(GB)

(74) Representative: Clark, Peter Frederick et al,
Imperial Chemical Industries Limited Legal Department,
Patents Thames House North Millbank
London SW1P 4QG(GB)

(54) **Process for the preparation of diphenylether carboxylic acids.**

(57) A process for the preparation of diphenyl ether carboxylic acids of the formula:

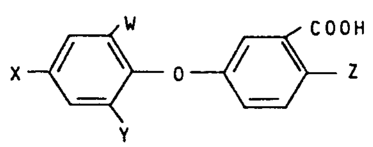

(111)

wherein W is hydrogen, fluorine, chlorine, bromine, iodine, trifluoromethyl or cyano; X is fluorine, chlorine, bromine, iodine or trifluoromethyl; Y is hydrogen, fluorine, chlorine, bromine, iodine or trifluoromethyl; and Z is hydrogen, fluorine, chlorine, bromine or iodine, which comprises oxidising a tolyl ether compound of the formula:

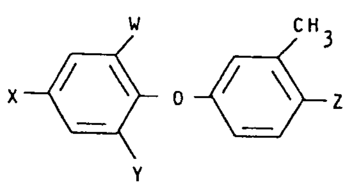

(iv)

wherein W, X, Y and Z have the meanings defined above, with molecular oxygen in the presence of a catalyst comprising a heavy metal compound and bromine or a bromine-containing compound.

EP 0 034 413 A1

This invention relates to a process for the preparation of diphenyl ether carboxylic acids which are themselves intermediates in the preparation of certain herbicides.

Published UK Patent Application No.2014565A describes and claims herbicidal diphenyl ether compounds of the formula (I), or a tautomer thereof.

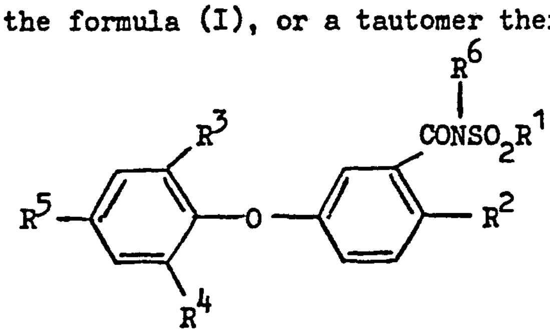

(I)

and salts thereof, wherein $R^1$ is an alkyl group of 1 to 6 carbon atoms optionally substituted by one or more fluorine atoms, or by a phenyl group optionally substituted by one or more halogen atoms; $R^2$ is a hydrogen atom, a fluorine, chlorine, bromine or iodine atom, or a nitro group; $R^3$ is a hydrogen atom, a fluorine, chlorine, bromine, or iodine atom, an alkyl group of 1 to 6 carbon atoms, a trifluoromethyl group, or a cyano group; $R^4$ is a hydrogen atom, a fluorine, chlorine, bromine, or iodine atom, or a trifluoromethyl group; $R^5$ is a fluorine, chlorine, bromine, or iodine atom or a trifluoromethyl group; and $R^6$ is a hydrogen atom or an alkyl group of 1 to 4 carbon atoms.

Also described are methods of preparing compounds of formula (I) starting from a carboxylic acid of the formula:

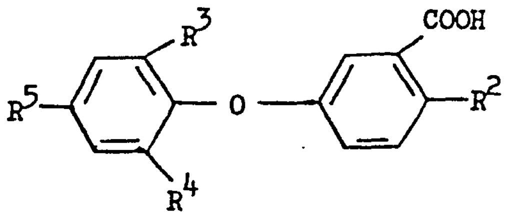

(II)

wherein $R^2$, $R^3$, $R^4$ and $R^5$ have the meanings given above.

According to the present invention there is provided a process for the preparation of diphenyl ether carboxylic acids of the formula:

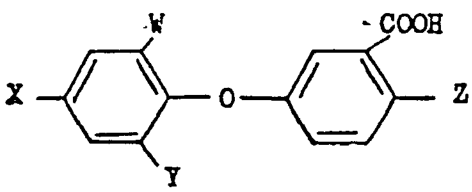

(III)

wherein W is hydrogen, fluorine, chlorine, bromine, iodine, trifluoromethyl or cyano; X is fluorine, chlorine, bromine, iodine or trifluoromethyl; Y is hydrogen, fluorine, chlorine, bromine, iodine or trifluoromethyl; and Z is hydrogen, fluorine, chlorine, bromine or iodine, which comprises oxidising a tolyl ether compound of the formula:

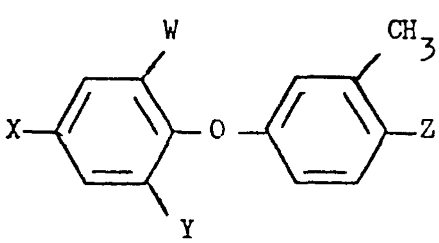

(IV)

wherein W, X, Y and Z have the previously defined meanings, with molecular oxygen in the presence of a catalyst comprising a heavy metal compound and bromine or a bromine-containing compound.

The molecular oxygen which is used in the process may be used alone or in admixture with other gases which are inert under the reaction conditions. Thus the oxygen may be used, for example, as air, as a mixture of oxygen and nitrogen with a higher or lower oxygen content than that of air, or as a mixture of oxygen and carbon dioxide. Particularly useful is a mixture of oxygen and nitrogen containing about 9% by volume of oxygen, which is safer to use than air when reactions are carried out under pressure.

The heavy metal compounds which may be used as components of the catalyst include compounds of vanadium, chromium, manganese, iron, cobalt, nickel, molybdenum, cerium and zirconium. Particularly suitable heavy metal compounds are compounds of cobalt and manganese, preferably used in combination.

Examples of the heavy metal compounds which may be used are cobalt (II) bromide, manganese (II) acetate, iron (III) chloride and nickel (II) bromide. Such compounds may be used in the anhydrous form but are more conveniently employed in the hydrated form containing water of crystallisation, i.e. as cobalt (II) bromide hexahydrate, manganese (II) acetate tetrahydrate, iron (III) chloride hexahydrate and nickel (II) bromide trihydrate respectively.

The catalyst comprises a heavy metal compound (or compounds) used together with bromine or a bromine-containing compound. The latter may be, for example, hydrogen bromide,

BAD ORIGINAL

an organic bromine compound such as tetrabromoethane or an inorganic bromide. Heavy metal compounds such as cobalt (II) bromide or manganese (II) bromide, by virtue of their bromine content, combine the function of heavy metal compound and bromine-containing compound, and may be used as the sole catalyst component, i.e. the separate addition of a bromine-containing compound is not essential. However it is preferred that the amount of bromine is in excess of the stoichiometric amount required to form a salt with the metal, especially up to 20:1 bromine:heavy metal (atomic ratio). Examples of inorganic bromides which may be used as bromine-containing compounds are hydrogen bromide and alkali metal or alkaline earth metal bromides such as the bromides of lithium, sodium, potassium, calcium and magnesium, and also ammonium bromide and quaternary ammonium bromides such as tetramethylammonium bromide.

The amount of heavy metal compound which is used is not critical, and may be, for example, from 0.01 to 20.0 mol %, preferably 0.1 to 10.0 mol %, of the tolyl ether compound starting material.

Preferred heavy metal components of the catalyst are mixtures of manganese and cobalt compounds. Particularly preferred is a manganese compound used together with a minor amount of a cobalt compound. For example, the cobalt compound may be employed in an amount of 0.05 to 0.5 atom of cobalt per atom of manganese. Such combinations are more economical to use than those in which a substantial amount of a cobalt compound is used, and are also more tolerant of aqueous reaction media.

BAD ORIGINAL

The process is preferably carried out in the presence of a solvent, which is desirably a mutual solvent for both the reactant of formula (IV) and the catalyst, and which should not be oxidised to any substantial extent during the reaction. Water may be used as the solvent but is most suitable when the reaction is carried out under pressure and at high temperatures because of the low aqueous solubility of the compounds of formula (IV). Other suitable solvents are aliphatic carboxylic acids, for example, glutaric acid, and preferably aliphatic monocarboxylic acids, especially those containing from 2 to 4 carbon atoms, e.g. acetic acid and propionic acid. Mixtures of such acids with water may be used. The amount of solvent is not critical, and may be from 2 to 100 times the weight of the reactant of formula (IV).

The reaction may be aarried out at normal atmospheric pressure and at temperatures from about 80°C up to the boiling point of the reaction mixture when water, an aliphatic monocarboxylic acid or a mixture of these is used as solvent. Under these conditions reaction times tend to be very prolonged. Thus, using cobalt (II) bromide as the sole catalyst and an aliphatic monocarboxylic acid containing from 2 to 4 carbon atoms such as acetic acid as solvent, reaction is extremely slow at the boiling point of the reaction mixture. A cobalt/manganese/bromide catalyst, for example, a mixture of cobalt (II) bromide and manganese (II) acetate gives an improved reaction rate, but even so the reaction may take up to 48 hours or even longer to go to completion. A significant part of this time is occupied by an induction period during which there

BAD ORIGINAL

is no detectable reaction. It has been found that the overall reaction time can be substantially reduced if the reaction is carried out in the presence of an oxidising agent in addition to molecular oxygen, for example, alkali metal, especially sodium and potassium, permanganates, bromates, chromates,dichromates, chlorates and perchlorates, and also chlorine and bromine. Paraldehyde is also an effective oxidising agent. The oxidising agent must be such that it does not inhibit reaction by interfering with the catalyst, for example, by causing the catalyst to precipitate and so removing it from the sphere of reaction.

Preferably the reaction is carried out in an autoclave under superatmospheric pressure and at temperatures up to 300°C, preferably 150-250°C. The pressure must be at least sufficient to maintain a liquid phase in the reaction vessel and will generally be within the range 1 to 20 bar, preferably 10 to 15 bar. At elevated temperature and pressure no induction period is observed and consequently no addition of oxidising agent other than oxygen is necessary.

The progress of the oxidation can be monitored, for example, by gas-liquid chromatographic (GLC) or high performance liquid chromatographic (HPLC) analysis of the reaction mixture, or conveniently by measurement of the oxygen content of the exit gases, which rises to a steady value at the completion of oxidation.

The product may be isolated from the reaction mixture by known means. Thus when water is the solvent, the product may simply be collected by filtration or by centrifuging from the cooled reaction mixture, washed with

BAD ORIGINAL

water and dried. If the solvent is non-aqueous, for example, acetic acid, the product may be precipitated from the cooled reaction mixture by dilution with a liquid which is miscible with the reaction solvent but which is a non-solvent for the product, or the non-aqueous reaction solvent may be removed, at least in part, by distillation or evaporation before addition of the non-solvent. The catalyst components are generally soluble in water, acetic acid and mixtures of these, and are thus readily removed from the product.

The invention is illustrated by the following Examples in which parts and percentages are by weight.

Example 1

2-Chloro-3'-methyl-4-trifluoromethyldiphenyl ether (175 parts), manganous acetate tetrahydrate (6 parts), cobaltous chloride hexahydrate (16 parts), potassium bromide (10 parts) and propionic acid (1000 parts) are stirred at 125-130°C. A steady stream of oxygen is introduced into the solution during 18 hours. After this time, the propionic acid is removed by distillation and water (1500 parts) is added to the residue with stirring. The resulting suspension is filtered and the residue is dissolved in a solution of sodium carbonate (64 parts) in water (1500 parts). The aqueous solution is extracted with toluene (172 parts) and the aqueous layer is acidified with concentrated hydrochloric acid (150 parts). The suspension so obtained is filtered and the solid residue is washed with cold water and dried at 60°C to give 3-(2-chloro-4-trifluoromethylphenoxy)benzoic acid [121 parts; strength 84% by high performance liquid chromatography (HPLC)].

BAD ORIGINAL

Example 2

2-Chloro-3'-methyl-4-trifluoromethyldiphenyl ether (175 parts), manganous acetate tetrahydrate (1.2 parts), cobaltous bromide hexahydrate (4.4 parts) and acetic acid (1000 parts) are stirred at 114-116°C. A steady stream of air is introduced into the solution during 48 hours. Oxidation products were detected by GLC after 22 hours. After completion of the reaction the acetic acid is removed by distillation and water (400 parts) is added with stirring. The resulting suspension is filtered and the solid residue is washed with cold water and dried at 60°C to give 3-(2-chloro-4-trifluoromethylphenoxy)benzoic acid (175 parts; strength 85% by HPLC; yield 77%).

A similar reaction which was stopped after 36 hours gave only a 44% yield of product.

Example 3

To acetic acid (1000 parts) is added manganous acetate tetrahydrate (1.2 parts), cobaltous bromide hexahydrate (4.4 parts) and potassium permanganate (0.7 part), and a stream of air is passed through the mixture. 2-Chloro-3'-methyl-4-trifluoromethyldiphenyl ether (175 parts) is then added and the mixture is stirred at 114-116°C. The induction period (i.e. period before oxidation products could be detected by GLC) was less than 30 minutes and the reaction was complete in 18 hours. Isolation of the product as described in Example 2 gave 3-(2-chloro-4-trifluoromethylphenoxy)benzoic acid (168 parts; strength 92% by HPLC; yield 80%).

BAD ORIGINAL

Example 4

The procedure described in Example 3 was repeated except that the 0.7 parts of potassium permanganate were replaced by 0.7 parts of potassium bromate. The induction period was less than 30 minutes. Reaction was continued for 24 hours to give 170 parts of product; strength 90% by HPLC; yield 79%.

Example 5

The procedure described in Example 3 was repeated except that the 0.7 part of potassium permanganate was replaced by 2.15 parts of bromine added before the air is introduced. The induction period was less than 30 minutes. Reaction was continued for 24 hours to give 170 parts of product; strength 92% by HPLC; yield 81%.

Example 6

2-Chloro-3'-methyl-4-trifluoromethyldiphenyl ether (286.5 parts), manganous acetate tetrahydrate (0.86 parts), cobaltous bromide hexahydrate (3.26 parts) and glacial acetic acid (1040 parts) were placed in a 5 cm. diameter x 85 cm. vertical titanium tube reactor, having a gas inlet and a liquid outlet at the base and equipped with a reflux condenser and with provision for electrical heating and water cooling. The reactor was pressurised to 13.8 bar (200 p.s.i.) with nitrogen and heated to 180°C. The nitrogen supply was disconnected and a mixture of 9% oxygen in nitrogen was passed into the solution via the bottom inlet at a rate equivalent to 9 l/minute at N.T.P.

The exit gases were monitored for oxygen content using a paramagnetic oxygen analyser and passage of gas was continued until the oxygen content of the exit gases

BAD ORIGINAL

showed a steady rise. This occurred after the oxygen containing gas had been passed into the reactor for 58 minutes.

Nitrogen was then passed into the reactor to displace residual oxygen, the reactor was cooled to 50°C and the pressure lowered to 2 bar.

The product in acetic acid solution was then run off via the base outlet valve of the reactor, the reactor was flushed out with acetic acid (200 parts) and these washings were added to the product solution.

HPLC analysis of the crude product solution indicated a 92% aonversion of the starting material to 3-(2-chloro-4-trifluoromethylphenoxy)benzoic acid.

Examples 7-12

The following preparations were carried out using the apparatus and general method of Example 6, but varying catalyst type and amount, reactant concentration, type of oxygen containing gas, solvent type (Example 11) and diphenylether starting material (Example 12). The products of Examples 7-11 were the same as in Example 6. The product of Example 12 was 3-(2-chloro-6-fluoro-4-trifluoromethylphenoxy)benzoic acid.

Details are shown in the following table.

BAD ORIGINAL

| Ex. No. | DIPHENYL ETHER (1) | | SOLVENT | | CATALYSTS (2) (parts) | | | | | GAS | | TEMP (°C) | PRESSURE (bar) | TIME (mins) | YIELD (%) (HPLC) |
|---|---|---|---|---|---|---|---|---|---|---|---|---|---|---|---|
| | Type | parts | Type | parts | Co | Mn | Fe | Ni | Br | Type | 1/min (NTP) | | | | |
| 7 | A | 286.5 | Acetic acid | 520 | 0.163 | 4.90 | - | - | 9.00 | Air | 13 | 190 | 13.8 | 23 | 91.4 |
| 8 | A | 286.5 | Acetic acid | 520 | - | 5.16 | - | - | 7.50 | 9% $O_2$ in $N_2$ | 8 | 190 | 13.8 | 103 | 82.3 |
| 9 | A | 286.5 | Acetic acid | 520 | - | - | 5.50 | - | 11.25 | 9% $O_2$ in $N_2$ | 6 | 190 | 13.8 | 180 | 14.1 |
| 10 | A | 286.5 | Acetic acid | 1040 | - | - | - | 5.50 | 6.0 | 9% $O_2$ in $N_2$ | 6 | 190 | 13.8 | 90 | 14.9 |
| 11 | A | 286.5 | Acetic acid + water | 520 + 200 | 0.654 | 4.90 | - | - | 7.50 | 9% $O_2$ in $N_2$ | 8 | 190 | 13.8 | 110 | 93.7 |
| 12 | B | 51.0 | Acetic acid | 730 | 0.06 | 0.80 | - | - | 1.50 | Air | 4 | 190 | 13.8 | 20 | 90.0 |

(1) A = 2-chloro-3'-methyl-4-trifluoromethyldiphenyl ether

B = 2-chloro-6-fluoro-3'-methyl-4-trifluoromethyldiphenyl ether.

(2) Co = $CoBr_2.6H_2O$ Fe = $FeCl_3.6H_2O$ Br = 48% w/w aq.HBr

Mn = $Mn(OAc)_2.4H_2O$ Ni = $NiBr_2.3H_2O$

CLAIMS

1. A process for the preparation of diphenyl ether carboxylic acids of the formula:

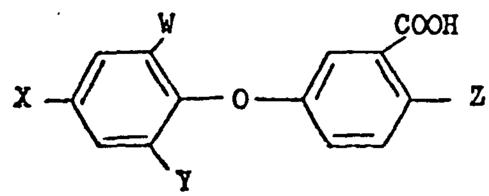

(III)

wherein W is hydrogen, fluorine, chlorine, bromine, iodine, trifluoromethyl or cyano; X is fluorine, chlorine, bromine, iodine or trifluoromethyl; Y is hydrogen, fluorine, chlorine, bromine, iodine or trifluoromethyl; and Z is hydrogen, fluorine, chlorine, bromine or iodine, which comprises oxidising a tolyl ether compound of the formula:

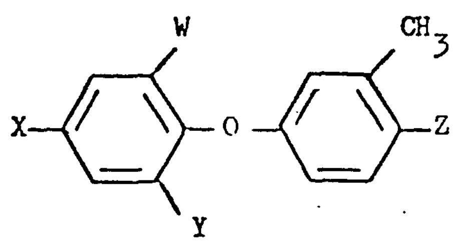

(IV)

wherein W, X, Y and Z have the meanings defined above, with molecular oxygen in the presence of a catalyst comprising a heavy metal compound and bromine or a bromine-containing compound.

2. A process as claimed in claim 1 wherein the heavy metal component of the catalyst comprises a compound of vanadium, chromium, manganese, iron, cobalt, nickel, molybdenum, cerium or zirconium.

BAD ORIGINAL

3. A process as claimed in claim 1 or claim 2 wherein the heavy metal component of the catalyst comprises a manganese compound, a cobalt compound, or both.

4. A process as claimed in claim 3 wherein the heavy metal component of the catalyst comprises a manganese compound and a cobalt compound in an amount of 0.05 to 0.5 atom of cobalt per atom of manganese.

5. A process as claimed in any one of claims 1 to 4 wherein the amount of heavy metal compound is from 0.01 to 20.0 mol % of the tolyl ether compound starting material.

6. A process as claimed in claim 5 wherein the amount of heavy metal compound is from 0.1 to 10.0 mol % of the tolyl ether compound starting material.

7. A process as claimed in any one of claims 1 to 6 wherein the component of the catalyst which is bromine or a bromine-containing compound is employed in amount up to 20:1 bromine:heavy metal (atomic ratio).

8. A process as claimed in any one of claims 1 to 6 wherein the heavy metal compound is itself a bromine-containing compound which is used as the sole catalyst component.

9. A process as claimed in any one of claims 1 to 8 wherein the reaction is carried out in a solvent which is water, an aliphatic monocarboxylic acid containing from 2 to 4 carbon atoms, or a mixture thereof.

10. A process as claimed in any one of claims 1 to 9 wherein the reaction is carried out at a pressure from 1 to 20 bar at least sufficient to maintain a liquid phase in the reaction vessel, and at a temperature up to 300°C.

BAD ORIGINAL

11. A process as claimed in claim 10 wherein the reaction is carried out at a pressure from 10 to 15 bar at least sufficient to maintain a liquid phase in the reaction vessel, and at a temperature from 150 to 250°C.

12. A process as claimed in any one of claims 1 to 9 wherein the reaction is carried out at normal atmospheric pressure in an aliphatic monocarboxylic acid containing from 2 to 4 carbon atoms as solvent, using a cobalt/manganese/bromide catalyst and in the presence of an oxidising agent in addition to molecular oxygen.

PFC/BH
20.1.81.

0034413

European Patent Office

# EUROPEAN SEARCH REPORT

Application number

EP 81 30 0284.7

## DOCUMENTS CONSIDERED TO BE RELEVANT

| Category | Citation of document with indication, where appropriate, of relevant passages | Relevant to claim |
|---|---|---|
| | EP - A1 - 0 002 749 (MATSUYAMA PETROCHEMICALS)<br>* claims 1, 7 *<br>-- | 1,3,4 |
| | GB - A - 1 496 821 (I.C.I.)<br>* claims 1, position b, 21 to 23 *<br>-- | 1-7 |
| | US - A - 3 674 845 (C. RENI et al.)<br>* claim 1 *<br>-- | 1 |
| | US - A - 3 970 696 (M. SHIGEYASU et al.)<br>* claims 1, 5, 6 *<br>-- | 1,3,4 |
| A | GB - A - 1 446 702 (ISHIHARA SANGYO KAISHA)<br>* claim 1 *<br>-- | 1 |
| A,P | Patents Abstracts of Japan, Vol. 4, No. 106, 30 July 1980 page 25C20<br>& JP - A - 55 - 66539<br>---- | 1 |

**CLASSIFICATION OF THE APPLICATION (Int. Cl.³)**

C 07 C 65/24
C 07 C 51/265
B 01 J 23/16
B 01 J 23/74
B 01 J 27/08

**TECHNICAL FIELDS SEARCHED (Int. Cl.³)**

B 01 J 21/06
B 01 J 23/10
B 01 J 23/16
B 01 J 23/34
B 01 J 23/74
B 01 J 27/08
C 07 C 51/265
C 07 C 65/21
C 07 C 65/24

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 26-05-1981 | KNAACK |